# EUROPEAN PATENT APPLICATION

(11) **EP 1 245 234 A1**
(43) Date of publication of application: **02.10.2002**
(21) Application number: 02380065.9
(22) Date of filing: 20.03.2002
(51) Int. Cl.: A61K 35/78, A61P 3/10

(54) **Procedure for obtaining the different fractions of atomised spanish guava and their use for the treatment of type II diabetes mellitus**

(30) Priority: 21.03.2001 ES 200100657
(71) Applicant: Laboratorio de Aplicaciones Farmacodinamicas, S.A., 08025 Barcelona (ES)
(72) Inventor: Osuna Carillo de Albornoz, Jose Ignacio, 18001 Granada (ES); Lopez Garcia, Maria Jose, Universidad de Granada, 18072 Granada (ES)
(74) Representative: Davila Baz, Angel

(57) **Abstract**

The present invention is related with new agents with hypoglucemic activity, derived from the Guava vegetable variety (Genus Psidium), with a procedure of obtaining such agents and with the use thereof in the preparation of a composition for the treatment of type II mellitus diabetes.

## Description

The present invention is related with new agents with hypoglucemic activity, derived from the Guava vegetable variety (Genus Psidium), with a procedure for obtaining such agents and with the use thereof in the preparation of a composition for the treatment of the type II diabetes mellitus.

The type II diabetes mellitus is a syndrome that is characterised by chronic hyperglucemia with an alteration of the metabolism of the carbohydrates, fats and proteins associated with an absolute or relative deficiency of the secretion of insulin and / or of the action of this hormone. As a consequence of the alteration in the production and / or action of the insulin, this clinical manifestation is accompanied by hyperglucemia, which is the agent responsible for the appearance of serious complications of diabetes mellitus like diabetic cataracts, retinopathy, microangiopathy, neuropathy and nephropathy.
For the prevention and the treatment of the type 2 diabetes mellitus, the use has been recommended of a balanced diet and physical exercise in a periodic way. Together with these therapeutic measures is the pharmacological treatment by means of oral antidiabetics. Basically the oral antidiabetics increase the levels of insulin released by the pancreatic B cell increasing in parallel the sensitivity of the peripheral tissue to insulin or diminishing the absorption of glucose at intestinal level, as is the case of the inhibitors of α-glucosidase or of the vegetable fibre.
The pharmacological treatment with antidiabetic compositions that act on the functionality of the pancreatic B cell and on the peripheral resistance to insulin, apart from some of them not managing to regulate the postprandial hyperglucemia peaks and others producing secondary effects or contraindications, with prolonged treatment, with these products, performance is gradually lost until possibly becoming ineffective. For this reason, some type 2 diabetics have to recur to treatment with insulin to control the glucemia levels.

Among the numerous treatments for type 2 diabetes, those most used are the oral antidiabetics that act on the pancreatic B cell and the peripheral resistance to insulin (biguanides, sulphonylureas, metformin, etc.) whose insulin secretor effect is due to their capacity to inhibit in a direct way by the ATP-regulated potassium channel. (*Ashford M.L.J., Sturgess N.C., Cook D.L., Hales C.N., Biophysics of the Pancreatic B-cell, I Atwater, E. Rojas and B Soria, eds., 1986. pp.* 69-76). These drugs being active at basal concentrations of glucose, can release insulin, under undesired conditions (at low concentrations of glucose) and cause severe hypoglucemia. For this reason alternative treatments have been proposed that might be capable of acting as a modulator in synergy with the glucose, that is to say, not being active for low concentrations of glucose (below the threshold level of 5-7 mM) whilst being active at higher concentrations.

The document US-A-4659715 makes known a method to reduce the corporal stores of fat using a prolactin inhibitor. However, this document states in column 2, lines 15 to 30 that a permanent administration is necessary of the prolactin inhibitor for the purpose of achieving the desired effects.

With this background, it is evident that it would be desirable to be able to have a new hypoglucemic agent that overcomes the aforementioned drawbacks in the state of the art.

In this sense, the applicant entity has carried out studies with the Guava vegetable variety of the Genus *Psidium*.
As a consequence of these studies it was possible to verify that certain fractions of Guava offer a solution for the treatment of type 2 diabetes mellitus, since not only did the symptoms of diabetes remit, but there was also a significant normoglucemic effect. This effect could be explained by the possibility that this treatment has a marked peripheral effect, diminishing specifically the peripheral resistance to the action of the insulin. This possibility leads to the idea that said effect was markedly different to that exercised by traditional oral antidiabetics like sulphonylureas and biguanides.

As a consequence, it was considered that the isolation of the molecule(s) responsible for this normoglucemic action, as well as the study of their action mechanism, can be of great interest as an alternative treatment for type 2 diabetes mellitus, with a prolonged action effect.

Therefore, according to a first aspect, the invention provides a new agent with hypoglucemic activity, characterised in that it comprises a fraction derived from the extraction with a solvent selected from the group consisting of methanol, butanol, hexane, chloroform and distilled water, of the Guava vegetable variety of the Genus Psidium, previously dehydrated.
In this first aspect of the invention, as Guava based on which the fractions are derived with hypoglucemic activity, the Spanish variety is preferably employed, without this implying the exclusion of other varieties of this plant. The same thing can be said in connection with other aspects of the invention described below.
According to a second aspect of the invention, a procedure is provided for obtaining fractions of Guava with hypoglucemic activity and responsible for a normoglucemic action in diabetic animals, characterised in that it comprises the steps of:
a) dehydrating the Guava;
b) subjecting the dehydrated Guava to extraction by employing methanol, hexane, or chloroform as solvents, in order to obtain thereby the different fractions of the Guava respectively;
c) isolate the methanolic, hexanic and chloroformic fractions so obtained, with hypoglucemic activity; and
d) subject the rest of the Guava to boiling in distilled water, in order to obtain after sedimentation the aqueous fraction with hypoglucemic activity.
In accordance with a modality of the procedure of the invention, the methanolic fraction obtained in step c) is dissolved in butanol in order to obtain thereby the butanolic fraction that likewise has hypoglucemic activity. This modality constitutes another characteristic of the procedure according to the invention.
As has been mentioned above, in the procedure of the invention the Spanish variety of the Guava plant is used preferably.
In accordance with the procedure according to the invention, the step of dehydrating the Guava can be carried out by atomisation making use of inulin or maltodextrin as support; or by liophilisation, preferably by atomisation.

It can be appreciated that the procedure of the invention comprises extracting the hydrosoluble and liposoluble constituents of the Guava, it being possible to carry out thereon subsequent fractioning processes with the object of purifying the component or the components responsible for the aforementioned normoglucemic action.

Details are provided below regarding the embodiment of the procedure according to the invention.

In step a) of dehydration by atomisation according to the procedure of the invention, the Guava is ground until a homogeneous paste is obtained. Next inulin or maltodextrin is added to prevent the caramelisation of the sugars in the fruit, it is pulverised and subjected to a current of hot air at 150-190° C, thereby evaporating the water present.
Step b) according to the procedure of the invention, started with Spanish Guava atomised with inulin or maltodextrin, in proportions of 30 to 70% of inulin or maltodextrin and 70 to 30% of Guava in dry condition.
As solvents for extracting the fractions, methanol, butanol, hexane, chloroform and distilled water were used.
For obtaining the fractions, a system was used of continuous extraction by means of 250 ml soxhlets.
120 g of atomised Guava with methanol, chloroform and hexane were placed in the soxhlets. After 20 to 28 hours had elapsed, the bath was set to the following temperatures for each solvent:
- Methanol: at 78 - 87° C (preferably at 82° C)
- Chloroform: at 70° C
- Hexane: at 78° C

Once the methanolic, hexanic and chloroformic fractions were extracted, the rest of the packet of atomised Guava was opened up and pulverised, being deposited in a beaker with boiling water, in the proportion of 1:8 by volume, and kept boiling for 15 minutes. After this time had elapsed, it was allowed to settle for a further 15 minutes, the aqueous fraction being obtained in this way by heating.

Once the methanolic fraction has been obtained, as has been indicated above, said fraction is dissolved in butanol to obtain the butanolic fraction that likewise has hypoglucemic activity.
For this, the methanolic fraction was dried completely in the rotavapor. The solid residue is resuspended in a mixture of butanol and water in a 1:1 ratio by volume. Afterwards in a decantation chamber the butanolic phase was separated in the upper and darker area, and the phase termed methanolic less the butanolic or M-B phase, in the lower and clearer area.
In accordance with a third aspect of the invention, the use was provided of the aforementioned fractions derived from the extraction with methanol, butanol, hexane, chloroform and water from the dehydrated Guava plant, in the preparation of a composition for the treatment of type 2 diabetes mellitus.
To determine the hypoglucemic effects of said fractions obtained according to the invention, male albino rats of the Wistar race were used, with a weight of between 250 and 300 g. The inducing of the diabetes was carried out by administering multiple subdiabetogenic doses of streptozotocin (30 mg/kg weight). It was considered that the animals had diabetes when the glucosuria they presented was greater than 500 mg/100 ml during at least 72 continuous hours.
To this end, the working plan that is described next was followed.

### Study of the hypoglucemic activity of the different fractions on the glucose homeostasis in diabetic rats

Once the fraction in question had dried, the solid residue was stored for not more than 72 hours at -20° C. Immediately before its administration to the diabetic rats, it was resuspended in the vehicle selected for its intraperitoneal administration.
In the case of the butanolic fraction, this together with the methanolic fraction, obtained in step d) of the procedure according to the invention, were dried in the rotavapor and moments before their administration they were reconstituted; the butanolic fraction in a 0.5 N solution of NaOH with a final pH of around 4; and the M-B fraction (see above) in distilled water
To evaluate the hypoglucemic activity of the Guava fractions obtained according to the invention, the glucemia of the animals was determined first (time 0). All the glucemia levels were determined by means of a glucose analyser that quantifies the concentration of hexose by the glucose oxidase method. This is an electrochemical technique using capillary suction. The blood sample is sucked toward the interior of the reagent tab by capillary action. The glucose in the blood reacts with the glucose oxidase and potassium ferricyanide present in the reactive area of the ribbon, forming potassium ferricyanide. The formation of potassium ferrocyanide is proportional to the concentration of glucose in the sample. During the oxidation, the potassium ferrocyanide produces an electric current that the analyser transforms into the glucose concentration.

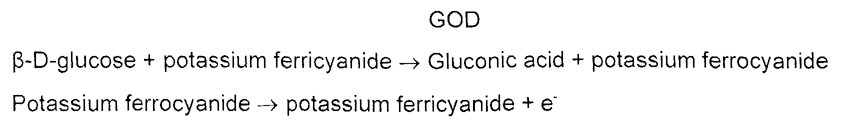

To obtain a blood sample, a small incision was made in the tail of the animal. Once the Guava extract had been administered intraperitoneally, the glucemia of the animal was determined at 6, 12, 24 and 36 hours after the treatment.

In these preliminary studies carried out with Guava atomised with inulin, the methanolic fraction was studied in comparison with the aqueous fraction. The basis for this decision was that in most of the studies on natural antidiabetics, the active principles isolated were for the most part hydrosoluble.

The methanolic fraction induced in the diabetic rats a decrease in the concentration of plasmatic glucose, a decrease occurring in the plasmatic levels of glucemia between 12 and 24 hours after the treatment.

Once the effect was known of the treatment with the methanolic fraction on the glucose homeostasis, the effect of the aqueous fraction obtained by heating was studied. It was possible to observe that the administration of this fraction, orally, induced a significant drop in the glucemia levels at 3 and 12 hours after its administration. It is noteworthy that the drop observed in glucemia levels was significantly less than that obtained with the treatment with the methanolic fraction.

Likewise and for comparative purposes, the treatment was carried out with the hexanic and chloroformic fraction, it being demonstrated that these produced a slight decrease of the glucemia levels in the diabetic rats at 6 and 12 hours after the treatment.
In contrast, the treatment with the methanolic fraction presented a marked hypoglucemic effect, maximum hypoglucemic activity being attained at 12 hours after its administration. As from that moment, the hypoglucemic action remained statistically significant (P = 0.004), until 48 hours after the treatment.

The intraperitoneal treatment with the butanolic fraction, induced a drop in the concentration of plasmatic glucose, starting 6 hours after its administration. From 12 to 48 hours both inclusive, after the treatment, the glucemia levels were normal.

### Study of the effect of the supports used in the dehydration by atomisation of Guava.

Once the diabetes was induced in the animals, the effect was studied of administering maltodextrin and inulin, in doses of 0.6 and 1 g. administered orally and intraperitoneally.

For this, once the basal glucemia was determined, the corresponding polysaccharide was administered, and starting from that point the glucemia was measured by reflectometry at 6, 12, 24 and 36 hours afterwards. It could be seen that the oral administration of 1 g of maltodextrin, produced no significant effect on the glucose homeostasis in the diabetic rats. On the contrary, if the concentration of this polysaccharide was reduced to 600 mg a significant decrease of the glucemia levels was observed, at 6 hours after its administration. From 12 hours until the end of the study, no significant differences were observed. This drop can be explained because said polysaccharide was not completely digested at intestinal level and the undigested remainder can produce stimulation of the enteroinsular axis, which would explain this decrease of the glucemia.
To discern whether the effect described was really due to a fibre effect or to some other mechanism, the experiment was repeated but with intraperitoneal administration of the maltodextrin, avoiding in this way the stimulation of the enteroinsular axis. It was observed that at both doses (0.6 and 1 g), intraperitoneal administration had a greater hypoglucemic effect than if it was administered orally. This confirmed the earlier observation that by incraperitoneal administration the effect arising from the digestion of the polysaccharide by the digestive enzymes is obviated.

The effect of the inulin was then studied when administered orally. It was verified that for a dose of 600 mg, the effect observed and commented above for the maltodextrin was reproduced. In the same way, when the dose was increased to 1 g, significant differences of the glucemia levels were not observed. When inulin was administered intraperitoneally, an identical effect was observed to that described for the maltodextrin. That is to say, for both doses studied, a significant drop was observed in the glucemia levels at 6 hours from their administration. Although starting from that time, the glucemia levels tended to recover, unlike that observed with the maltodextrin, at 12 hours from the inulin administration, a significant drop of the glucemia was still observed with respect to the basal values.

Of all the results presented up to now, the methanolic and butanolic fractions were those that presented the most marked antidiabetic activity, it being possible to state that both fractions have normoglucemic activity.

### Dose of Guava extract administered in each of the fractions studied.

Of the fractions studied of Spanish Guava, the inulin concentration was determined in each of them. The purpose of carrying out this determination, was to know the quantity of Guava that was being administered and thereby to calculate the dose in each treatment.

The method selected for the inulin determination was by means of colorimetric analysis, as described by Führ, J. Kaczmarczyk and C.D. Krüttgen, slightly modified. This method consists of a prior deproteinisation of the sample with trichloroacetic and the subsequent determination of the concentration of fructose coming from the inulin by colorimetry, at a wavelength of 620 nm. The quantity of inulin present in each of the fractions, was expressed in the number of grams extracted, that is to say according to the yield obtained. The yield of the fractions obtained in each of the extractions of the atomised Spanish Guava, is given in the following table:

| FRACTION | GRAMS |
|---|---|
| Hexanic | 1.5-3 |
| Chloroformic | 0.2-1.5 |
| Methanolic | 18-38 |
| Butanolic | 2.65-10.34 |
| M-B | 16-29.35 |
| Aqueous | 7.19-19.48 |

In the following table gives the doses of inulin and Guava administered intraperitoneally:

| FRACTION | INULIN | GUAVA |
|---|---|---|
| Hexanic | - | 0.2-1.5 |
| Chloroformic | - | 0.14-0.72 |
| Methanolic | 0.26-1.14 | 0.2-1.43 |
| Butanolic | 0.08-0.34 | 0.71-2.34 |
| M-B | 0.8-2.4 | 0.61-3.2 |
| Aqueous | 0.43-1.35 | 0.33-1.74 |

The values of inulin of the methanolic, M-B and aqueous fractions appear in boldface because their real value is unknown. This is because, due to their polarity, the fructose content of these fractions is significant and the method of determining the inulin is based on the reaction of the fructose released from the inulin molecule with the anthron for which reason crossed reactions appear.

As was expected, in the hexanic and chloroformic fraction, inulin did not appear. In these fractions only those compounds were found that are apolar and therefore liposoluble. The inulin is a fructose polymer and therefore very hydrosoluble, for which reason it should not be found in the fractions intended for extraction of the liposoluble compounds.

Of the other fractions studied, the most apolar solvent is butanol. This means that the inulin percentage in this fraction will be minimal.

The hypoglucemic effects indicated above are illustrated in the attached drawings wherein:

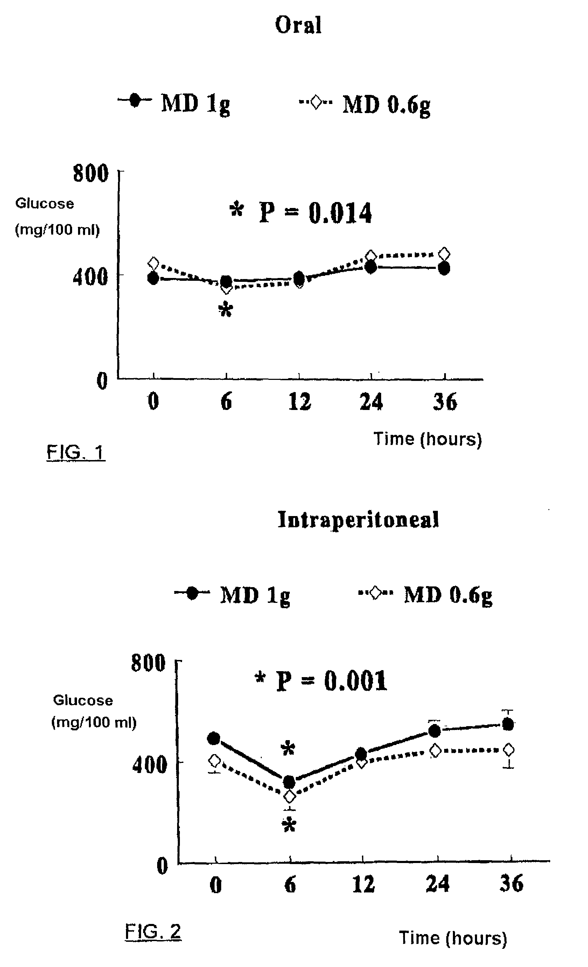

**Figure 1** shows the effect of oral administration of maltodextrin, 1 or 0.6 g.
**Figure 2** shows the effect of intraperitoneal administration of maltodextrin.

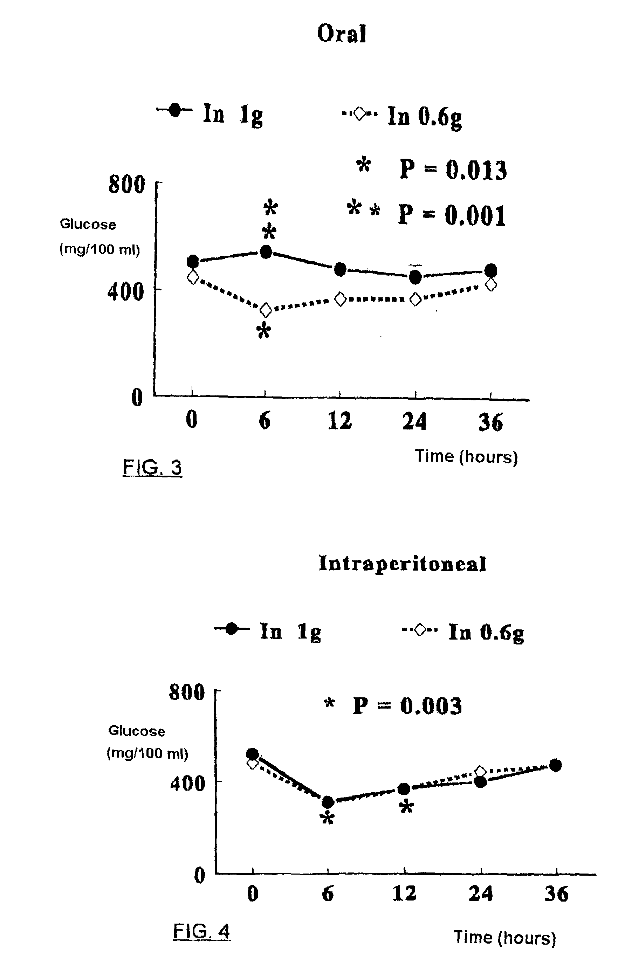

**Figure 3** shows the effect of oral administration of 0.6 and 1 g of inulin.
**Figure 4** shows the effect of intraperitoneal administration of 0.6 and 1 g of inulin.

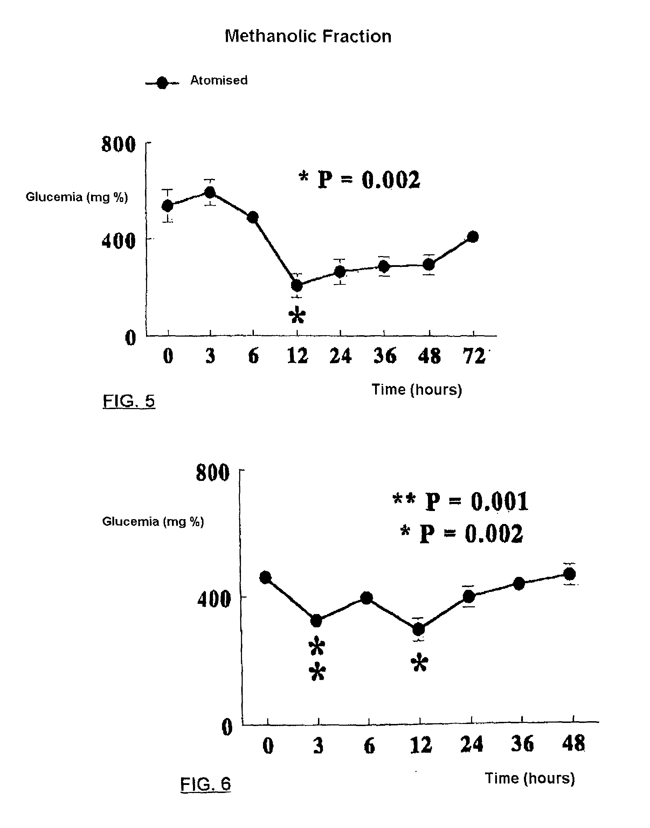

**Figure 5** shows the effect of the treatment with the methanolic fraction obtained from Spanish Guava atomised with inulin in the proportion of 60/40, demonstrating how in all the animals treated a drop is observed in the levels of plasmatic glucose.
**Figure 6** shows the effect of treatment with the aqueous fraction obtained by heating Spanish Guava atomised with inulin in the proportion of 60/40, by oral administration.

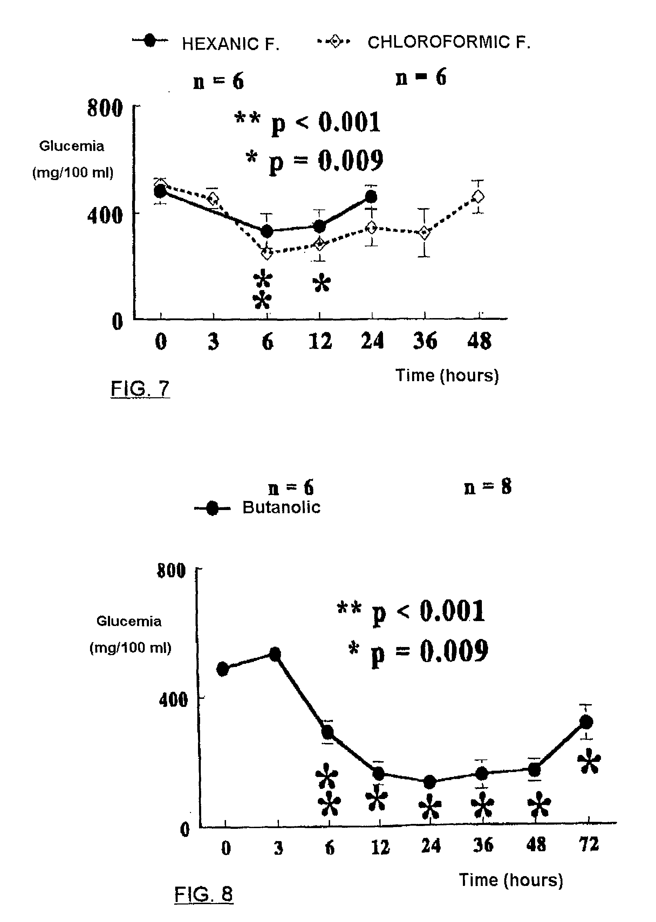

**Figure 7** shows the effect of intraperitoneal treatment with the hexanic fraction (full line with filled circles) and the chloroformic fraction (broken line with unfilled diamonds) obtained from Spanish Guava atomised with inulin, 60/40.
**Figure 8** shows the effect with the butanolic fraction of Spanish Guava on the glucose homeostasis in diabetic rats. The number of rats studied was 8.

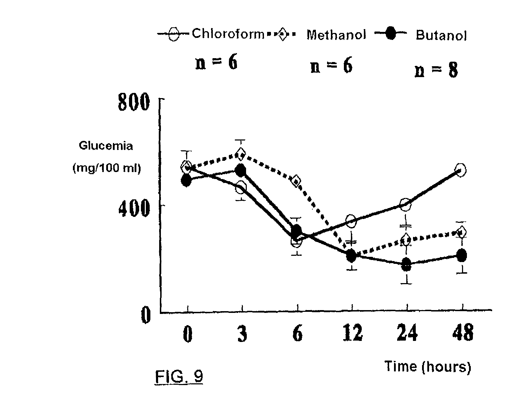

**Figure 9** shows the drop in the concentration of plasmatic glucose during the 48 hours following treatment with the chloroformic (n=6), methanolic (n=6) and butanolic (n=8) fraction.
   *P = 0.043
   **P < 0.001

## Claims

1. An agent with hypoglucemic activity, **characterised in that** it comprises a fraction derived from the extraction with a solvent chosen from the group consisting in methanol, butanol, hexane, chloroform and distilled water, of the Guava vegetable variety of the genus Psidium, previously dehydrated.

2. An agent with hypoglucemic activity, according to claim 1, **characterised in that** the Guava vegetable variety is the Spanish variety.

3. A procedure for obtaining fractions of Guava with hypoglucemic activity and responsible for a normoglucemic action in diabetic animals, **characterised in that** it comprises the steps of:
a) dehydrating the Guava;
b) subjecting the dehydrated Guava to extraction by employing methanol, hexane or chloroform as solvents, in order to obtain thereby the different fractions of the Guava respectively,
c) isolating the methanolic, hexanic and chloroformic fractions so obtained, with hypoglucemic activity; and
d) subjecting the rest of the Guava to boiling in distilled water, in order to obtain after sedimentation the aqueous fraction with hypoglucemic activity.

4. A procedure according to claim 3, **characterised in that** as Guava the Spanish variety of Guava of the genus Psidium is used.

5. A procedure according to claim 3, **characterised in that** in step (a), the dehydration is carried out by atomisation making use of inulin or maltodextrin as support.

6. A procedure according to claim 3, **characterised in that** in step (a), the dehydration is carried out by atomisation making use of liophilisation.

7. A procedure according to any of claims 3 to 6, **characterised in that** the methanolic fraction obtained in step c) is dissolved in butanol to obtain thereby the butanolic fraction which likewise has hypoglucemic activity.

8. Use of the agent with hypoglucemic activity according to claims 1 and 2 in the preparation of a pharmaceutical composition for the treatment of type 2 diabetes mellitus.

9. Use according to claim 8, **characterised in that** the methanolic fraction is employed.

10. Use according to claim 8, **characterised in that** the butanolic fraction is employed.
